(19) Europäisches Patentamt
European Patent Office
Office européen des brevets

(11) **EP 1 462 125 B1**

(12) **EUROPEAN PATENT SPECIFICATION**

(45) Date of publication and mention
of the grant of the patent:
**18.06.2008 Bulletin 2008/25**

(51) Int Cl.:
***A61L 24/10*** *(2006.01)*      ***A61L 24/00*** *(2006.01)*

(21) Application number: **04251849.8**

(22) Date of filing: **29.03.2004**

(54) **Adhesive antineoplastic compositions**

Selbstklebende antineoplastische Zusammensetzung

Composition anti-néoplastique adhésive

(84) Designated Contracting States:
**AT BE BG CH CY CZ DE DK EE ES FI FR GB GR HU IE IT LI LU MC NL PL PT RO SE SI SK TR**

(30) Priority: **28.03.2003 US 457924 P**

(43) Date of publication of application:
**29.09.2004 Bulletin 2004/40**

(73) Proprietor: **ED. GEISTLICH SÖHNE AG FÜR CHEMISCHE INDUSTRIE**
**6110 Wolhusen (CH)**

(72) Inventors:
• **Stendel, Rüdiger**
**14163 Berlin (DE)**
• **Pfirrmann, Rolf W.**
**6006 Lucerne (CH)**

(74) Representative: **Marsden, John Christopher**
**Frank B. Dehn & Co.**
**St Bride's House**
**10 Salisbury Square**
**London EC4Y 8JD (GB)**

(56) References cited:
**US-A1- 2001 031 870      US-A1- 2002 111 345**
**US-A1- 2002 131 935**

• **SEMPLE J E ET AL: "Potent and selective thrombin inhibitors featuring hydrophobic, basic P3 ?P4-aminoalkyllactam moieties" BIOORGANIC & MEDICINAL CHEMISTRY LETTERS, OXFORD, GB, vol. 8, no. 24, 15 December 1998 (1998-12-15), pages 3525-3530, XP004819386 ISSN: 0960-894X**

## Description

The present invention is in the field of antineoplastic compositions.

Taurolidine [bis-(1,1-dioxoperhydro-1,2,4-thiadiazin-4-yl)-methane] was developed by Geistlich Pharma. It is a white crystalline substance, water soluble up to 2%. It is made up of two molecules of taurinamide and three molecules of formaldehyde forming a two-ringed structure bridged by a methylene group.

Taurolidine primarily has antibiotic and anti-endotoxin effects. It acts by a chemical reaction, so that no microorganism resistance to it has as yet been observed. These effects of taurolidine are mediated by its active metabolites, methylol-taurultam and methylol-taurinamide, which are donors of active methylol groups; these groups operate by reacting with the cell walls of bacteria and with the primary amino groups of endotoxins.

Additional effects of taurolidine which have been reported include: inhibition of tumour necrosis factor and interleukin-1β in mononuclear cells (Bedrosian 1991), inhibition of tumour necrosis factor toxicity, and inhibition of peritoneal tumour cell growth in laparoscopic surgery (Jacobi 1997).

Taurolidine solutions have been used for instillation to or rinsing of the abdominal cavity in cases of peritonitis. In post-operative instillations, some conscious patients have reported irritation as a side-effect, sometimes together with burning sensations.

Monson et al. in PCT International Publication Number WO 92/00743 disclose a selective direct inhibiting effect of taurolidine and/or taurultam on certain body tumours. Preliminary evidence that taurolidine, as well as being anti-endotoxin and antimicrobial, is also antineoplastic on B16 melanoma cells and Meth A sarcoma cells in a mice model *in vivo*, and on fibroblastic tumour cells, LS174T (colon) carcinoma cells and Jurkat (leukaemic) cells *in vitro* is reported by Monson JRT, Ramsey PS, Donohue JH; Abstract. Br. J. Surg. 77(6), 1990, A 711.

US Patent Application Publication No. 2002/O111345 discloses that tumour growth in a mammal may be inhibited through administration of taurolidine, taurultam or a biologically active derivative thereof, e.g. orally, intravenously, by infusion into the brain or cerebrospinal fluid, or by way of an implantable erodible or resorbable solid matrix such as a wafer or sponge. Therapeutic compositions such as ointments, pastes, sprays, patches (including adhesive patches), creams, gels, resorbable sponges and foams containing these active ingredients are also disclosed.

In systemic chemotherapy, antineoplastic agents are unspecifically distributed throughout the body *via* circulation. Proliferating cells in healthy organs are thus exposed to the same concentrations of the agent as are tumour cells. Moreover, intratumoural distribution of the agent may be prevented by different haemodynamic factors in the tumour. The antineoplastic action of most chemotherapeutic agents depends on the difference in proliferation rates between normal cells and tumour cells; when these rates are the same, dose-limiting adverse events may occur. It is generally assumed that the effectiveness of chemotherapy increases with the concentration of the agent within the tumour and the duration of exposure. On the other hand, systemic administration is limited by the severity of adverse events.

One approach to overcome this problem is to administer chemotherapeutic agents locally, relying on diffusion for their distribution. In local therapy, the antineoplastic agent is introduced into the tumour itself or into the area around it. The resulting pressure gradient leads to diffusion of the antineoplastic agent into the tumour. This mode of administration not only increases the concentration of the agent within the tumour but also results in much lower concentrations in other tissues compared to systemic administration.

US Patent Application Publication No. 2002/0131935 discloses "compounds" for treatment of cancer which may be applied to the margins of a tumour removal site and which comprise a fibrin carrier and a therapeutic agent intermixed therewith. Representative agents are said to include radionuclides and chemotherapy agents such as alkylating agents, nitrogen mustard, antimetabolites, antitumour antibiotics, plant alkaloids, hormones and hormone antagonists.

Various materials such as collagen or biodegradable polymers or silicones may be used in local drug delivery systems, including systems for the local delivery of antineoplastic agents. The materials may serve as matrices by means of which embedded local cytostatic agents such as bischloroethylnitrosourea, mitoxantrone or cisplatin may be introduced into a tumour resection cavity. Potential problems with this mode of drug administration may arise when the carrier matrix contains components that undergo complete degradation only after a very long time or not at all. Another risk is that of uncontrolled distribution of the antitumour agent in the cerebrospinal fluid, which may make it difficult accurately to determine the concentration at the target. Postoperative changes in the shape and size of a tumour resection cavity associated with oedema formation may preclude complete filling of the cavity with carrier matrices such as drug-carrying wafers. The resulting inhomogeneous distribution of the agent may lead to pronounced local increases in drug concentration which may have toxic effects on adjacent healthy tissue.

Another approach to local tumour treatment is so-called convection-enhanced drug delivery in which the drug is infused into the tumour or surrounding tissue and is then distributed by convective transport. However, this mode of administration requires placement of a catheter in most cases, which increases the risk of infection and the incidence of postoperative cerebrospinal fluid fistula formation. Furthermore, it is better suited to cases of non-resected tumours and can only be applied with difficulty following tumour resection.

Accordingly there remains a need in the art for new methods and compositions for treating tumours.

In accordance with the present invention there is provided an antineoplastic composition comprising taurolidine, taurultam or a mixture thereof suspended at a concentration of 20-160 mg/ml in a biodegradable adhesive comprising a fibrin sealant capable of adhering to tissue of a living subject.

The invention further provides the use of taurolidine, taurultam or a mixture thereof and a biodegradable adhesive comprising a fibrin sealant capable of adhering to tissue of a living subject in the manufacture of a medicament wherein said taurolidine, taurultam or mixture thereof is suspended in said biodegradable adhesive at a concentration of 20-160 mg/ml, and wherein said medicament is for application as an antineoplastic, for example in preventing or inhibiting growth of cancer cells, in particular by inducing death of neoplastic cells through apoptosis.

Taurolidine and the related product taurultam have been shown to exert a modifying effect on the toxicity of tumour necrosis factor which is used, *inter alia*, in the treatment of tumours. Furthermore, their action has been shown to be selective in that the growth of normal cell-lines is not significantly inhibited.

Taurolidine acts by transferring three methylol groups at the site of action, taurultam being an intermediate metabolite which itself transfers a single methylol group with liberation of the very well tolerated compound taurinamide and ultimately taurine. Taurine is an amino acid that is present in the body in large quantities, especially in the heart and in the brain.

It should be noted that methylol transfer is to be distinguished from methyl transfer, the latter being characteristic of many highly toxic anti-tumour drugs. Methylol transfer agents such as taurolidine and taurultam, on the other hand, have low toxicity and are not cytotoxic against normal cells.

Cancers to which compositions of the present invention may be applicable include recurrent glioblastoma, glioma, neuroblastoma, astrocytoma, carcinomatous meningitis, ovarian cancer, prostate cancer, central nervous system cancer, lung cancer, gastric cancer, oesophageal cancer, urinary bladder cancer, leukaemia, mesothelioma, lymphoma, melanoma, renal cell cancer and metastases thereof. Other cancers against which compositions of the present invention may be effective include other carcinomas, sarcomas or lymphomas, cancers of the head and neck, liver cancer, breast cancer and pancreatic cancer.

Treatment of central nervous system cancers, as well as inhibition of tumour metastases thereof, are particularly important applications of the compositions.

In one preferred embodiment of the invention, the compositions are applied following at least partial extirpation of primary and/or secondary brain tumours or other tumours of the central nervous system. Other preferred applications are in the treatment of skin tumours, tumours in the mouth/jaw/face region, squamous cell carcinoma, tumours in the genitourinary area, tumours of the outer eye and the eyelids, bone tumours, tumours of the parenchymal organs, and tumours of the gastrointestinal tract.

In another preferred embodiment, a composition of the invention is applied as a layer to an area of tissue after at least partial removal of a tumour therefrom, preferably by spraying or brushing the composition onto the surface area of the cavity resulting from removal of the tumour. The thus-formed layer may advantageously have a thickness of about 0.1-10 mm, preferably about 1-5 mm, and most preferably about 1.5-2.5 mm.

The term biodegradable adhesives as used herein is intended to encompass bioabsorbable or erodible adhesives.

The compositions of the invention are initially in a fluid or semi-fluid state, more preferably a liquid or a semi-liquid state, when applied and adhered to an area of tissue, *e.g.* from which a tumour has been removed. The adhesive then increases in viscosity or at least partially solidifies following application and while adhering to the tissue.

The concentration of taurolidine and/or taurultam in the compositions is preferably 50-80 mg/ml.

The adhesive utilised in the present invention is a fibrin sealant, also known as fibrin glue. Fibrin glue is a two-component system of separate solutions of fibrinogen and thrombin/calcium. When the two solutions are combined, the resultant mixture mimics the final stages of the clotting cascade to form a fibrin clot, referred to hereinafter as a fibrin sealant matrix. The fibrinogen component can be prepared extemporaneously from autologous, single-donor or pooled blood. Fibrin glue is available in Europe under the brand names Beriplast, Tissel and Tissucol, and has been used in a wide variety of surgical procedures to repair, seal and attach tissues in a variety of anatomic sites.

In particularly preferred usages, after application of a layer of a composition according to the invention over an area to be treated, the layer is covered and sealed with a sealing second layer which does not contain taurolidine or taurultam. The sealing second layer can comprise the same biodegradable adhesive utilised in the composition of the invention, for example a fibrin sealant.

Malignant gliomas tend to recur in the vast majority of cases. Recurrent gliomas may arise from vital tumour cells present in the zone around the resection margin. Recurrent gliomas grow very rapidly, and quickly result in death of the patient. The compositions of the present invention may be used to combine tumour resection with local chemotherapy using an antineoplastic but non-toxic agent. Taurolidine exerts a selective antineoplastic effect by induction of programmed cell death, and has anti-angiogenic activity. Fibrin sealant is completely degradable and firmly adheres to brain tissue, thereby providing a matrix for taurolidine delivery - a taurolidine-fibrin sealant matrix - in the local treatment of brain tumours.

Taurolidine and/or taurultam may be suspended homogeneously in both the thrombin and the procoagulant protein components of the fibrin sealant. The fibrin sealant matrix subsequently formed following combination of the components is a suitable carrier for the suspension of taurolidine and/or taurultam at concentrations which ensure the release of therapeutically effective amounts of the drug over a period of up to two weeks or longer, as confirmed by *in vitro* tests. The antineoplastic action of taurolidine is not affected by embedding in the fibrin sealant matrix.

Thus the compositions of the invention may be suitable for interoperative local treatment of brain tumours, *e.g.* with taurolidine, either following complete tumour resection or following partial resection of tumours which are not fully resectable because of their location.

Malignant gliomas invade surrounding tissue and therefore tend to recur in the vast majority of cases even after apparently complete gross resection. Gliomas recur within 2 cm of the original resection margin in 80-90% of cases. Thus, recurrent gliomas may arise from vital tumour cells present in this zone around the resection margin. The extent of tumour resection correlates with postoperative survival. Furthermore, metastases from malignant gliomas are very rare and primarily extracerebral in location.

In accordance with one application of compositions of the invention, total or partial tumour resection is combined with local chemotherapy using an agent such as taurolidine which exhibits selective antineoplastic activity without damaging normal brain tissue. In accordance with one embodiment, such drug delivery into the brain enables close contact with the tumour or the walls of the resection cavity, and allows the delivery of the drug in therapeutically effective concentrations with minimal toxic effects on healthy brain tissue. Moreover, the delivery is devoid of the risk of infection and leaves no residue which might cause local complications.

Additionally, since the presence of taurine in the brain is abnormally low in glioblastoma patients, administration of taurolidine and/or taurultam has the additional advantage of raising the taurine level in the brain, since both compounds are ultimately metabolised into taurine.

As noted above, taurolidine has a double effect against tumours, triggering the induction of programmed tumour cell death (apoptosis) and also having anti-angiogenic activity by inhibiting vascular endothelial growth factor and transforming growth factor-beta. It may therefore be appropriate to employ simultaneous intravenous administration of a methylol transfer agent such as taurolidine in patients with glioblastoma in combination with administration of compositions according to the invention.

Example

Fibrin sealant matrices were prepared using Tissel kits (Immuno AG, Vienna, Austria, provided by Baxter Deutschland GmbH, Heidelberg, Germany) according to manufacturer's instructions. Taurolidine (ultrapure) was provided by Geistlich Pharma AG, Wolhusen, Switzerland.

Different concentrations of taurolidine were homogenously suspended in the two components of the fibrin sealant. The two components were then mixed in wells of a 24-multiwell plate to prepare matrices with identical total volumes of 400 μl per well containing final taurolidine concentrations of 10, 20, 40 and 80 mg/ml. The resulting thickness of the matrix was 2 mm ± 0.4 mm. In addition, matrices with volumes of 400 μl and 800 μl containing 10 mg/ml or 40 mg/ml of taurolidine were prepared to investigate the effect of matrix size on taurolidine release. The 800-μl matrix had a thickness of 4mm ± 0.3 mm. Matrices of identical volume and thickness without taurolidine served as controls. Supernatants of 400 μl phosphate-buffered saline were added to each well after solidification. The multiwell plates were incubated at 37°C. The supernatants were pipetted off at 24-hour intervals over a period of 7 days and replaced by identical amounts of fresh phosphate-buffered saline.

The long-term release kinetics of taurolidine were investigated by homogeneously suspending different concentrations of taurolidine in the two components of the fibrin sealant in such a way that final concentrations (mg/ml) of 10, 40 and 80 mg/ml were achieved per 400 μl of fresh phosphate-buffered saline at 24-hour intervals over a period of 14 days. The supernatants from the wells containing identical taurolidine concentrations were pooled. The taurolidine concentrations in the supernatants were determined.

To determine whether the antineoplastic activity of taurolidine was affected by embedding in the matrix, the glial tumour cell lines LN 18, LN229, U87MG, and *ex vivo* cells from a freshly isolated glioblastoma were incubated with taurolidine released from the matrices at different periods.

The LN18, LN229, and U87MG tumour cells and *ex vivo* cells from a glioblastoma were seeded in 150 cm$^3$ plastic cell culture flasks until a cell confluency of 80% was reached. The resulting cell suspensions were centrifuged at 1200 rpm for 5 minutes and then diluted to yield cell suspensions containing 5 x 10$^4$ cells per ml. Aliquots of 200 μl of the cell suspensions were pipetted into the wells of a 96-multiwell plate.

After 12 hours when the cells were adherent the medium was removed and replaced by fresh medium. The cells were incubated for 24 hours and the taurolidine solution released at different time intervals from the matrices loaded with different taurolidine concentrations. Tumour cells incubated with the same volume of supernatant from fibrin sealant matrix without taurolidine were used as negative controls. Identically treated cells to which Fas-ligand at a concentration of 25% was added served as positive controls.

After incubation for 24 hours, the supernatants were removed and 100 μl of crystal violet staining solution (0.5% crystal violet in 19.5% methanol and 80% distilled water) were added. The solution was removed after 10 minutes and residual dye rinsed off with tap water. The plates were then left to air-dry for 12 hours followed by counting in a microplate counter at 540 nm.

The theoretical assumptions underlying the diffusion-controlled release of taurolidine from the fibrin sealant matrix were reviewed. Using a model of local taurolidine metabolism, the factors affecting the diffusion-controlled release of taurolidine from the matrix were investigated.

The two components of the fibrin sealant containing the suspended taurolidine were sprayed through a single nozzle at 1.5 bar by means of filter-sterilised compressed air. The highest possible concentration of taurolidine in the resulting matrix was investigated. It was determined whether a uniform distribution of the taurolidine-fibrin sealant matrix could be achieved.

The investigation of taurolidine release from the fibrin sealant matrix over a period of 1 week showed an exponential increase of the cumulative amount of taurolidine released.

The cumulative amounts of taurolidine released into the supernatant at different concentrations of taurolidine loading differed statistically significantly [analysis of variance, Kruskal-Wallis-test; $p < 0.001$]. The exponential course of cumulative taurolidine release from the matrix resulted in the largest amounts of taurolidine being released within the first days.

The temporal course of percentage taurolidine release was determined in relation to the initial taurolidine load of the matrix. The results suggested that, irrespective of the initial taurolidine concentration, about 50% of the total taurolidine was released from the matrix within the first 2 days ($54.7 \pm 1.44\%$) and about 75% within 6 days ($75.2 \pm 6.64\%$). The percentage release rates did not differ significantly for the different taurolidine loading concentrations in the taurolidine-fibrin sealant matrix (analysis of variance, Kruskal-Wallis-test; $p = 0.522$).

Cumulative taurolidine release differed significantly for constant concentrations of taurolidine but different matrix volumes (analysis of variance and Holm-Sidak-test; $p < 0.001$) while no statistically significant differences in cumulative taurolidine release were seen for identical initial amounts of taurolidine in different matrix volumes (analysis of variance and Holm-Sidak-test; $p = 0.934$ (initial amount of 8 mg) and $p = 0.159$ (initial amount of 32 mg).

The loaded amount of taurolidine in the matrix seems to be the crucial determinant of release. Apparently, the thickness of the matrix does not have an important role in controlling taurolidine release. This observation is crucial for the practical application of the fibrin sealant matrix since it may not be possible ensure a uniform matrix thickness in all cases.

A long life span of the taurolidine-fibrin sealant matrix is desirable to ensure local therapy over an adequate period of time. The life span of a fibrin matrix is limited by the onset of fibrinolysis. The data available so far suggest that the *in vivo* life span can at most be extended to 12 - 14 days when an antifibrinolytic such as aprotinin is added. Moreover, the rate at which the fibrin sealant matrix is degraded varies with the proteolytic activity at the site of application.

We therefore investigated whether taurolidine is released throughout the maximum life span of the matrix of 14 days. In the experimental model used here, antineoplastically effective amounts of taurolidine were released throughout the 14-day observation period at the loading concentrations of taurolidine to 25 mg/ml, or above. Loading concentrations of 50 mg/ml or higher resulted in the release of over 100 μg/ml of taurolidine on day 14. This taurolidine level is above the ECso of acute cytotoxicity for most of the cell lines tested with taurolidine before.

The temporal course of taurolidine release was investigated for different loading amounts of taurolidine in the matrix over a period of 14 days. Irrespective of loading concentrations, $98.93\% \pm 0.33$ of the taurolidine had been released from the matrix after 10 days and almost 100% ($99.99\% \pm 0.02$) after 14 days. The percent release did not change for the different loading concentrations of taurolidine (analysis of variance, Kruskal-Wallis test; $p = 0.830$).

Proliferation of all the tumour cell lines investigated, and of the *ex vivo* glioblastoma cells, was inhibited in an concentration-dependent manner. Taurolidine released on day 13 from taurolidine-fibrin sealant matrix with an initial taurolidine loading concentration of 10 mg/ml had no further effects on tumour cell proliferation. This is in accordance with the concentration of taurolidine released from the matrix at this time. In contrast, taurolidine released on day 3 with a loading concentration of 100 mg/ml was found to reduce cell counts by at least 60%.

The taurolidine-loaded fibrin sealant matrix was applied intraoperatively after removal of a brain tumour in such a way as to ensure close binding to the wall of the cavity and filling out all the surface irregularities. Taurolidine then diffused from the matrix compartment into the brain compartment along the diffusion gradient, which is the main force underlying taurolidine release. At the same time, the process of elimination of taurolidine from the brain area adjacent to the matrix starts; by metabolism, on one hand, and by diffusion into deeper brain areas, on the other hand.

In addition to these processes, taurolidine may enter and get distributed in the cerebrospinal fluid space. This distribution is driven by diffusion through the matrix surface facing the resection cavity and enhanced by cerebrospinal fluid convection, particularly when the resection cavity remains "open". To counteract these undesired losses, a multilayer matrix was applied, with the top layer comprising fibrin sealant without taurolidine. This extra layer reduced the loss of drug due to diffusion into and convection of cerebrospinal fluid. Losses of drug by the route were taken into consideration by using a safety factor in calculating taurolidine delivery.

The taurolidine-fibrin sealant matrix undergoes fairly little dissolution or erosion and therefore releases the suspended drug it carries primarily by diffusion. The release is dominated by the diffusion velocity of the drug from the matrix into surrounding tissue. The release rates under these conditions are in linear relationship to the square root of time.

The amount of taurolidine released from the matrix is directly proportional to the diffusion area. Therefore, a proportionality factor for the different initial concentrations of taurolidine in the matrix is determined, which then can be used to calculate taurolidine release as a function of time and diffusion area.

The experimental results and the calculated data showed no statistically significant differences for any of the initial taurolidine concentrations investigated.

Taurolidine-fibrin sealant matrix releases of taurolidine were sufficient in concentration to exert antitumour activity over 14 days. The results of the *in vitro* experiments suggest that an initial taurolidine concentration of 80 mg/ml is appropriate.

The required matrix volume was calculated from the amount of taurolidine released and the initial taurolidine concentration, $C_o$, according to the equation:

$$V_{TFM} = \frac{M\left(1 + k_s\right)}{C_o},$$ where $V_{TFM}$ = required taurolidine-fibrin sealant matrix volume [ml]; M = cumulative amount of taurolidine released [mg]; $k_s$ = safety factor; $C_o$ = initial taurolidine concentration in the matrix [mg/ml]. The safety factor was introduced to compensate for diffusion losses into the fibrin sealant cover layer and subsequent losses by convention. The safety factor chosen was 1.

The taurolidine-fibrin sealant was applied to the resection cavity by spraying. Application by spraying facilitates even distribution of the resulting matrix on the walls of the resection cavity. Suspension of taurolidine in the two components of the fibrin sealant and spraying of the drug delivery system posed no problems for initial taurolidine loading concentrations of up to 80 mg/ml. It was possible to apply the sealant very homogeneously and even in multiple layers due to the short coagulation time.

The limitations of prior art local drug administration can be overcome by use of compositions comprising taurolidine and/or taurultam and a biodegradable adhesive comprising a fibrin sealant as disclosed herein. Such a sealant may be sprayed over the walls of a resection cavity after total or partial tumour resection; during coagulation fibrinogen will be converted into fibrin and will form covalent bonds with surrounding proteins, resulting in a layer of haemostatically active sealant matrix which will subsequently be degraded by proteolytic activity. This matrix has haemostatic effects such as are necessary or desirable after surgery. Additionally, covalent bonding to surrounding proteins ensures that the matrix stays exactly where it has been applied. This is crucial since the displacement of brain structures after tumour resection and postoperative oedema formation may change the size and the shape of the resection cavity.

The experimental results demonstrate that the taurolidine is released from the taurolidine-fibrin sealant matrix under infinite-sink conditions over 2 weeks in concentrations that have definitive antineoplastic effects on the tumour cell lines and *ex vivo* tumour cells investigated here. A study investigating the release of antibiotics from a fibrin matrix suggests that the limited-sink model is most suitable to describe the *in vivo* conditions. The experiments using different tumour cell lines and *ex vivo* glioblastoma cells show that the antitumour activity of taurolidine is not affected by its embedding in the fibrin sealant matrix.

Taurolidine is known to have no cytotoxic effects on normal cells. Earlier cell culture experiments using neuronal and glial brain cells obtained from rat foetuses on day 15 of gestation showed that taurolidine has no cytotoxic effects on these cells. A taurolidine concentration of 80 mg per ml of fibrin sealant an be homogenously suspended in the matrix.

A fibrin sealant matrix is a suitable carrier for the suspension of taurolidine at a concentration that ensures the release of therapeutically effective amounts of the drug over a period of 2 weeks *in vitro.* The antineoplastic action of taurolidine is not affected by embedding in and release from fibrin sealant matrix. Higuchi's model of drug release from matrices provides a suitable approximation for describing the diffusion-controlled release of taurolidine from the fibrin sealant matrix.

The described drug delivery system is suitable for local taurolidine treatment of brain tumours following complete or partial resection or of tumours that are non-resectable because of their location.

**Claims**

1. An antineoplastic composition comprising taurolidine, taurultam or a mixture thereof suspended at a concentration of 20-160 mg/ml in a biodegradable adhesive comprising a fibrin sealant capable of adhering to tissue of a living subject.

2. A composition as claimed in claim 1 wherein said concentration is 50-80 mg/ml.

3. A composition as claimed in claim 1 or claim 2 wherein said fibrin sealant comprises separate thrombin and procoagulant protein components, and said taurolidine, taurultam or mixture thereof is suspended in both of said components.

4. Use of taurolidine, taurultam or a mixture thereof and a biodegradable adhesive comprising a fibrin sealant capable of adhering to tissue of a living subject in the manufacture of a medicament wherein said taurolidine, taurultam or mixture thereof is suspended in said biodegradable adhesive at a concentration of 20-160 mg/ml, and wherein said medicament is for application as an antineoplastic.

5. Use as claimed in claim 4 wherein said medicament is for application to an area of tissue from which a tumour has been removed.

6. Use as claimed in claim 4 or claim 5 wherein said medicament is for application by spraying.

7. Use as claimed in any of claims 4 to 6 wherein said medicament is for application as a layer.

8. Use as claimed in claim 7 wherein said medicament is for application as a layer having a thickness of 0.1-10 mm.

9. Use as claimed in claim 8 wherein said medicament is for application as a layer having a thickness of 1-5 mm.

10. Use as claimed in claim 9 wherein said medicament is for application as a layer having a thickness of 1.5-2.5 mm.

11. Use as claimed in any of claims 7 to 10 wherein said layer of medicament is to be covered and sealed with a second sealing layer which does not contain taurolidine or taurultam.

12. Use as claimed in any of claims 5 to 11 wherein said tumour is a glioblastoma.

**Patentansprüche**

1. Anti-neoplastische Zusammensetzung, die Taurolidin, Taurultam oder ein Gemisch davon einer Konzentration von 20 - 160 mg/ml in einem biologisch abbaubaren Klebstoff, der ein Fibrin-Dichtmittel aufweist, suspendiert aufweist, die in der Lage ist, an Gewebe eines lebenden Individuums zu haften.

2. Zusammensetzung wie in Anspruch 1 beansprucht, bei der die Konzentration 50 - 80 mg/ml beträgt.

3. Zusammensetzung wie in Anspruch 1 oder 2 beansprucht, bei der das Fibrin-Dichtmittel getrennte Trombin- und koagulationsfördernde Protein-Komponenten aufweist, und das Taurolidin, Taurultam oder Gemisch davon in beiden der Komponenten suspendiert ist.

4. Verwendung von Taurolidin, Taurultam oder eines Gemisches davon und eines biologisch abbaubaren Klebstoffs, der ein Fibrin-Dichtmittel aufweist, der in der Lage ist, an Gewebe eines lebenden Individiums zu haften, zur Herstellung eines Arzneimittels, in dem das Taurolidin, Taurultam oder Gemisch davon in dem biologisch abbaubaren Klebstoff in einer Konzentration von 20 - 160 mg/ml suspendiert sind, und wobei das Arzneimittel ein Arzneimittel zur Anwendung als ein anti-neoplastisches Mittel ist.

5. Verwendung wie in Anspruch 4 beansprucht, bei der das Arzneimittel ein Arzneimittel zur Auftragung auf einem Gewebebereich, von dem ein Tumor entfernt wurde, ist.

6. Verwendung wie in Anspruch 4 oder Anspruch 5 beansprucht, bei der das Arzneimittel ein Arzneimittel zur Auftragung durch Sprühen ist.

7. Verwendung wie in irgendeinem der Ansprüche 4 bis 6 beansprucht, bei der das Arzneimittel ein Arzneimittel zur Auftragung als eine Schicht ist.

8. Verwendung wie in Anspruch 7 beansprucht, bei der das Arzneimittel ein Arzneimittel zur Auftragung als eine Schicht mit einer Dicke von 0,1 - 10 mm ist.

9. Verwendung wie in Anspruch 8 beansprucht, bei der das Arzneimittel ein Arzneimittel zur Auftragung als eine Schicht mit einer Dicke von 1 - 5 mm ist.

10. Verwendung wie in Anspruch 9 beansprucht, bei der das Arzneimittel ein Arzneimittel zur Auftragung als eine Schicht mit einer Dicke von 1,5 - 2,5 mm ist.

11. Verwendung wie in irgendeinem der Ansprüche 7 bis 10 beansprucht, bei der die Schicht des Arzneimittels mit einer zweiten Dichtschicht, die nicht Taurolidin oder Taurultam enthält, zu Bedecken und Abzudichten ist.

12. Verwendung wie in irgendeinem der Ansprüche 5 bis 11 beansprucht, bei der der Tumor ein Glioblastom ist.

**Revendications**

1. Composition anti-néoplastique comprenant de la taurolidine, du taurultam ou un mélange de ceux-ci en suspension à une concentration de 20 à 160 mg/ml dans un adhésif biodégradable comprenant une colle à base de fibrine capable d'adhérer aux tissus d'un sujet vivant.

2. Composition selon la revendication 1, dans laquelle ladite concentration est de 50 à 80 mg/ml.

3. Composition selon la revendication 1 ou 2, dans laquelle la colle à base de fibrine comprend de la thrombine et des composants protéiques procoagulants distincts, et lesdits taurolidine, taurultam ou mélange de ceux-ci est en suspension dans ces dits deux composants.

4. Utilisation de la taurolidine, du taurultam ou d'un mélange de ceux-ci et d'un adhésif biodégradable comprenant de la colle à base de fibrine capable d'adhérer aux tissus d'un sujet vivant dans la fabrication d'un médicament dans lequel lesdits taurolidine, taurultam ou mélange de ceux-ci sont en suspension dans ledit adhésif biodégradable à une concentration de 20 à 160 mg/ml, et dans laquelle ledit médicament est destiné à une application comme anti-néoplastique.

5. Utilisation selon la revendication 4, dans laquelle ledit médicament est destiné à une application sur une zone de tissu de laquelle une tumeur a été enlevée.

6. Utilisation selon la revendication 4 ou 5, dans laquelle ledit médicament est destiné à une application par pulvérisation.

7. Utilisation selon l'une quelconque des revendications 4 à 6, dans laquelle ledit médicament est destiné à une application sous forme de couche.

8. Utilisation selon la revendication 7, dans laquelle ledit médicament est destiné à une application sous forme de couche ayant une épaisseur de 0,1 à 10 mm.

9. Utilisation selon la revendication 8, dans laquelle ledit médicament est destiné à une application sous forme de couche ayant une épaisseur de 1 à 5 mm.

10. Utilisation selon la revendication 9, dans laquelle ledit médicament est destiné à une application sous forme de couche ayant une épaisseur de 1,5 à 2,5 mm.

11. Utilisation selon l'une quelconque des revendications 7 à 10, dans laquelle ladite couche de médicament doit être couverte et scellée avec une seconde couche de scellement qui ne contient pas de taurolidine ou de taurultam.

12. Utilisation selon l'une quelconque des revendications 5 à 11, dans laquelle ladite tumeur est un glioblastome.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9200743 A, Monson
- US 2002O111345 A

- US 20020131935 A

**Non-patent literature cited in the description**

- **MONSON JRT ; RAMSEY PS ; DONOHUE JH.** *Abstract. Br. J. Surg.,* 1990, vol. 77 (6), A 711